# EUROPEAN PATENT APPLICATION

(11) **EP 3 323 871 A2**
(43) Date of publication of application: **23.05.2018**
(21) Application number: 16824763.3
(22) Date of filing: 15.07.2016
(51) Int. Cl.: C09K 11/06, C07D 209/82, C07D 495/04, C07D 401/04, C07D 403/04, H01L 51/00, H01L 51/50

(54) **ORGANIC HOLE TRANSPORTING COMPOUND USING P-DOPED CONJUGATED POLYMER ELECTROLYTE, ORGANIC ELECTRONIC DEVICE USING SAME, AND MANUFACTURING METHOD THEREFOR**

(30) Priority: 15.07.2015 KR 20150100314
(71) Applicant: Konkuk University Industrial Cooperation Corp., Gwangjin-gu Seoul 05029 (KR)
(72) Inventor: MOON, Doo Kyung, Seoul 06670 (KR); CHOI, Min-Hee, Bucheon-si Gyeonggi-do 14774 (KR); LEE, Eui Jin, Seongnam-si Gyeonggi-do 13462 (KR)
(74) Representative: Schiweck, Weinzierl & Koch Patentanwälte Partnerschaft mbB
(86) International application number: PCT/KR2016/007757
(87) International publication number: WO 2017/010847

(57) **Abstract**

An organic hole transporting compound and an organic photovoltaic device comprising the same are provided. The organic hole transporting compound has a P-doped structure and an SO₃⁻ functional group at the end of the main chain of a conjugated polymer, and thus has excellent solubility in alcohol-based solvents. Also, the organic hole transporting compound does not exhibit an acidity, and thus can implement highly-efficient hole mobility with excellent long-term stability, and can be effectively applied to organic photovoltaic devices, etc.

## Description

### [Technical Field]

The present invention relates to a conjugated polymer organic electrolyte in which an SO₃⁻ functional group is introduced to the main chain thereof and an organic photovoltaic device employing the same as a hole transport layer, and more particularly, to a conjugated polymer organic electrolyte having a P-doped structure.

### [Background Art]

As high oil prices and environmental pollution have become issues, research on low-priced environmentally-friendly energy sources is being actively performed, and among the research, the most noticeable field is organic photovoltaic devices (OPVs). The OPV refers to a device in which an exciton is formed as a photon or a pair of electron and a hole in an organic active layer receiving sunlight by means of an organic photovoltaic (PV) phenomenon, and moves along the interface between an electron donor material and an electron acceptor material, and is separated due to a LUMO level difference, thereby producing electricity.

The OPV is a thin film-type device with a simple structure, which is light-weight so as to be portable, can be manufactured in a low-cost process, and is flexible. Particularly, a variety of research to overcome low energy conversion efficiency through the improvement in device structure as well as the development of a novel material is progressing. In terms of a manufacturing process, a polymer and an oligomer do not need a vacuum deposition process and are prepared in a simple process through a solution process, and therefore are suitable for mass production and production of a large-sized device.

To implement polymer and oligomer photovoltaic devices enabling such a solution process, a variety of research is progressing, but these devices still exhibit lower efficiency than solar cells produced by a deposition process. One of the various reasons for this is the introduction of various electron transport layers and hole transport layers through a deposition process. Among these, these days, a PEDOT:PSS polymer is most widely used as a material for hole transport layers of an organic light emitting device and an organic solar cell and also applied in improvement of a device structure due to advantages of solution processability and water solubility. However, the PEDOT:PSS has been reported to have serious quenching of excitons occurring at the interface with an active layer, and may have adverse effects on the life span and efficiency of the device by oxidization of a positive electrode due to strong acidity. Therefore, to replace PEDOT:PSS, there is a demand for research and development of a polymer hole transport layer enabling an alcohol solution process, which allows stacking through the solution process and does not affect the morphology of an active layer. However, conjugated polymers have poor solubility in alcohols due to the rigid main chain thereof.

### [Disclosure]

### [Technical Problem]

The present invention is directed to providing a P-doped organic electrolyte which has excellent hole transfer capability and enhances energy conversion efficiency of an organic solar cell and an OPV employing the same as a hole transport layer.

### [Technical Solution]

In one aspect, the present invention provides an organic hole transporting compound including a repeat unit represented by Formula 1 below.

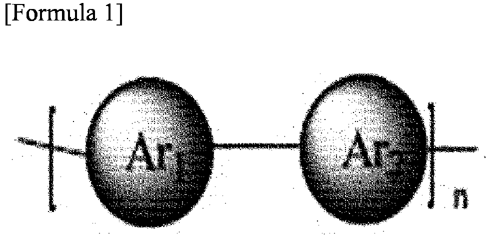

In Formula 1,
Ar₁₋ is represented by Formula 2 below,
Ar₂ is represented by Formula 3 below, and
n is an integer of 1 to 1,000,000.

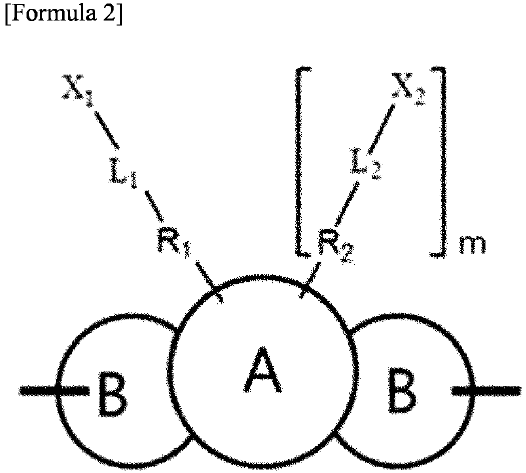

In Formula 2,
A is a C3 to C16 cyclic structure, in which any one or more of the carbons forming the cyclic structure A are substituted with N or Si, or not substituted,
B is a ring structure which is linked to the cyclic structure A at both ends,
L₁ and L₂ are each independently a single bond, a C1 to C6 alkylene, C6 to C16 arylene or C3 to C15 heteroarylene structure,
R₁ and R₂ are each independently any one selected from the group consisting of a single bond, a double bond, -O-, C1 to C6 alkylene; C1 to C6 alkoxylene; thiophene; thiophene substituted with a C1 to C25 alkyl group; thiophene substituted with a C1 to C25 alkoxy group; selenophene; selenophene substituted with a C1 to C25 alkyl group; selenophene substituted with a C1 to C25 alkoxy group; pyrrole; pyrrole substituted with a C1 to C25 alkyl group; pyrrole substituted with a C1 to C25 alkoxy group; an arylene group; an arylene group substituted with a C1 to C25 alkyl group; an arylene group substituted with a C1 to C25 alkoxy group; an aryl group; an aryl group substituted with a C1 to C25 alkyl group; an aryl group substituted with a C1 to C25 alkoxy group; thiazole; thiazole substituted with a C1 to C25 alkyl group; thiazole substituted with a C1 to C25 alkoxy group; and a C10 to C24 aryl group having a fused ring aromatic compound,
X₁ and X₂ are each independently SO₃⁻, SO₃⁻M⁺, N(CH₃)₂, N(CH₃)₃⁺Br⁻ or N(C₂H₄OH)₂,
M is H, K, Na or Li, and
m is 0 or 1.

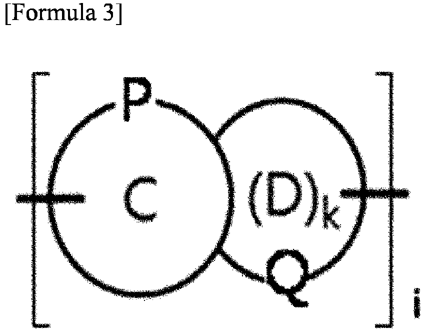

In Formula 3,
C and D are each independently a C3 to C20 ring structure, and include an element represented as P or Q,
k is an integer of 0 or 1, and when k is 0, it represents that there is no ring structure represented by D,
i is an integer of 1 or 2, and
P and Q are each independently any one selected from the group consisting of carbon, oxygen, sulfur and selenium.

In another aspect, the present invention provides an OPV including the above-described organic hole transporting compound.

### [Advantageous Effects]

An organic hole transporting compound according to the present invention has an SO₃⁻ functional group at ends of the main chain of a conjugated polymer, excellent solubility in an alcoholic solvent and does not exhibit acidity, is able to implement highly efficient hole mobility with long-term stability, and thus can be effectively applied to OPVs, etc.

### [Description of Drawings]

FIGS. 1A to IF show the results of measuring absorbances of UV-Vis spectra for precursors prepared in Preparation Examples 1 to 9.
FIGS. 2A to 2E are UV absorption and PL spectra for compounds represented by Formula 5 according to the present invention.

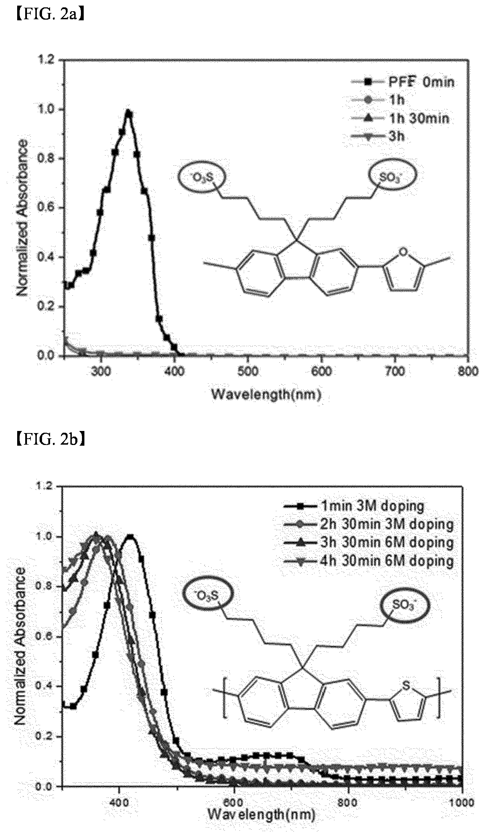

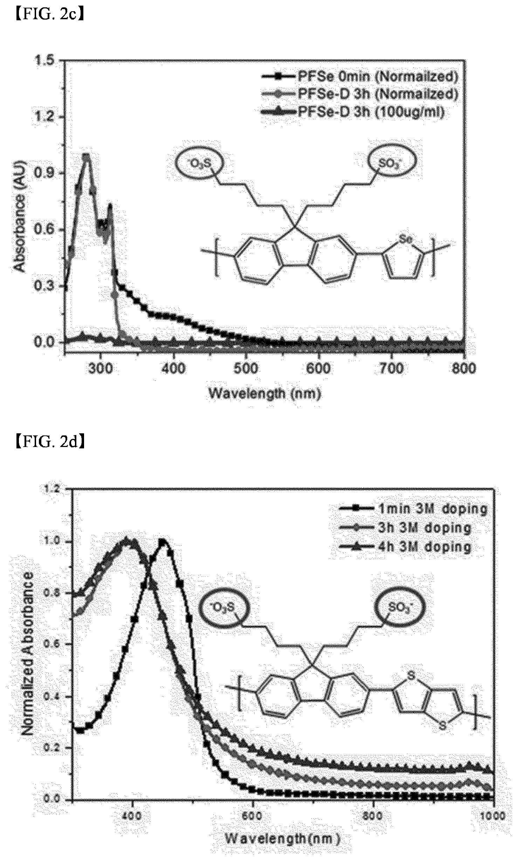

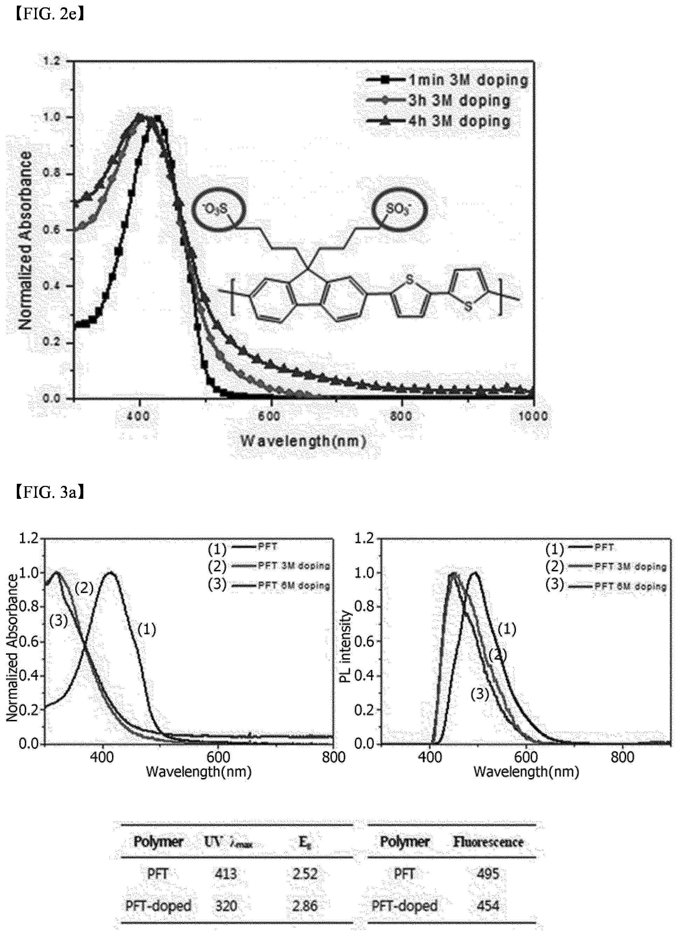

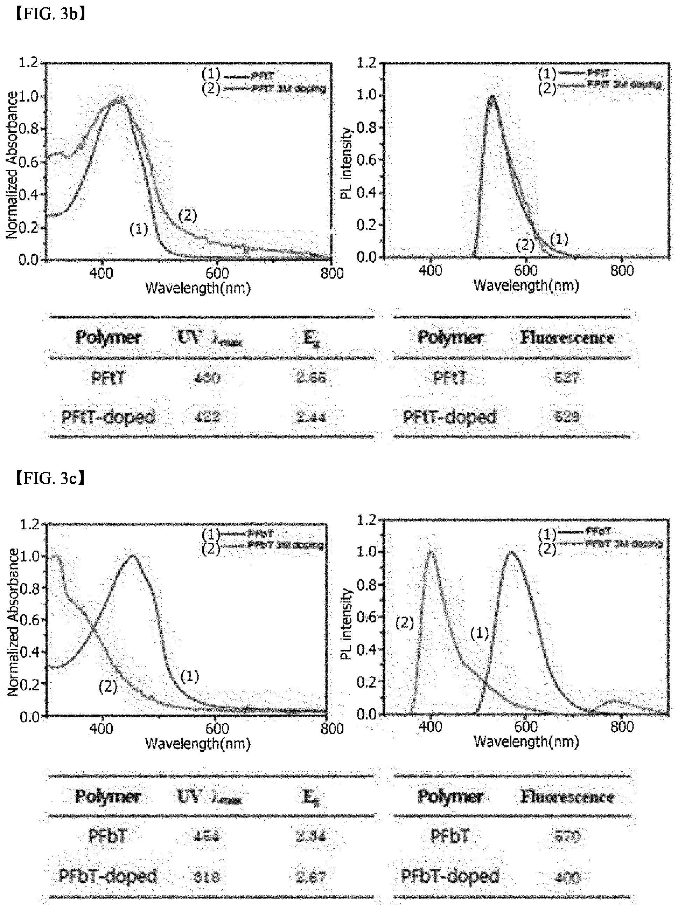

FIGS. 3A to 3C show absorbance graphs (left). While FIGS. 2B, 2D and 2E show the change in absorption regions to check doping degrees of PFT, PFtT and PFbT, the absorbance graphs of FIGS. 3A to 3C are provided to compare absorbance of compounds represented by Formula 4, such as PFT, PFtT and PFbT, and compounds represented by Formula 5, such as PFT-D, PFtT-D, PFbT-D, after collection of the completely-doped PFT-D, PFtT-D, PFbT-D polymers. Since the absorption regions of PFT-D, PFtT-D and PFbT-D are shorter wavelength regions (regions that contain less photon energy from sunlight reaching the earth surface) than those of PFT, PFtT and PFbT, these regions can transmit most of the sunlight through a photoactive layer, and are preferable for energy harvesting.
FIG. 4 is the cyclic voltammetry (CV) graph for evaluating an electrochemical characteristic of the compound represented by Formula 5 according to the present invention and the band diagram for the compounds.
FIG. 5 shows work function values measured using UPS by spin-coating an ITO glass with PFF, PFT, PFSe, PFtT, PFbT, and PFT-D, PFtT-D and PFbT-D doped with these polymers.
FIGS. 6A to 6B show the current-voltage curves for photovoltaic devices in which each of the compounds represented by Formulas 4 and 5 according to the present invention and PEDOT:PSS as a comparative example is introduced as a hole transport layer.
FIGS. 7A and 7B show the results of measuring external quantum efficiency (EQE) when each of the compounds represented by Formula 4 and the polymers corresponding to Formula 5, which are doped with the polymers of Formula 4, is applied to an organic solar cell as a hole transport layer (HTL).
FIGS. 8A and 8B show long-term stability test results for organic solar cells using polymers according to the present invention and an organic solar cell using PEDOT:PSS.
FIG. 9 is a graph of comparing the voltage-current density of organic solar cells according to Example 19 of the present invention.
FIG. 10 shows the result of evaluating the long-term stability of the organic solar cells according to Example 19 of the present invention.

### [Modes of the Invention]

In one exemplary embodiment, an organic hole transporting compound includes a repeat unit represented by Formula 1 below.

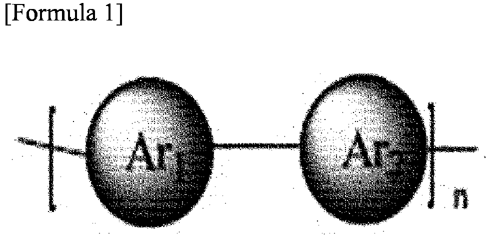

In Formula 1,
Ar₁₋ is represented by Formula 2 below, Ar₂ is represented by Formula 3 below, and n is an integer of 1 to 1,000,000.

First, Ar₁ may be represented by Formula 2 below.

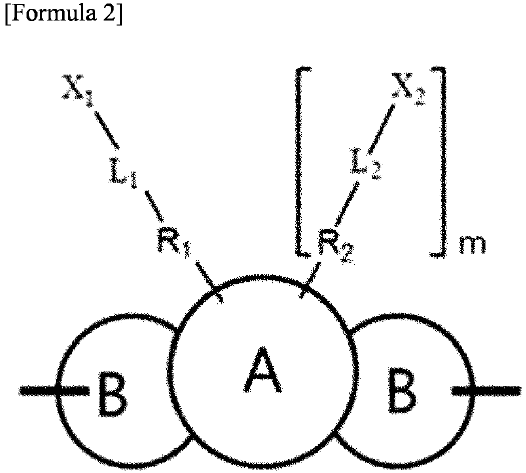

In Formula 2,
A is a C3 to C16 cyclic structure, in which any one or more of the carbons forming a cyclic structure A are substituted with N or Si, or not substituted,
B is a ring structure which is linked to the cyclic structure A at both ends,
L₁ and L₂ are each independently a single bond, a C1 to C6 alkylene, C6 to C16 arylene or C3 to C15 heteroarylene structure,
R₁ and R₂ are each independently any one selected from the group consisting of a single bond, a double bond, -O-, C1 to C6 alkylene; C1 to C6 alkoxylene; thiophene; thiophene substituted with a C1 to C25 alkyl group; thiophene substituted with a C1 to C25 alkoxy group; selenophene; selenophene substituted with a C1 to C25 alkyl group; selenophene substituted with a C1 to C25 alkoxy group; pyrrole; pyrrole substituted with a C1 to C25 alkyl group; pyrrole substituted with a C1 to C25 alkoxy group; an arylene group; an arylene group substituted with a C1 to C25 alkyl group; an arylene group substituted with a C1 to C25 alkoxy group; an aryl group; an aryl group substituted with a C1 to C25 alkyl group; an aryl group substituted with a C1 to C25 alkoxy group; thiazole; thiazole substituted with a C1 to C25 alkyl group; thiazole substituted with a C1 to C25 alkoxy group; and a C10 to C24 aryl group having a fused aromatic compound,
X₁ and X₂ are each independently SO₃⁻, SO₃⁻M⁺, N(CH₃)₂, N(CH₃)₃⁺Br⁻ or N(C₂H₄OH)₂,
M is H, K, Na or Li, and
m is 0 or 1.

More specifically, in the definition of Formula 2,
A is a C3 to C16 cyclic structure, in which any one or more of the carbons forming the cyclic structure A are substituted with N or Si, or not substituted,
B is a ring structure which is linked to the cyclic structure A at both ends,
L₁ and L₂ are each independently a single bond or a C1 to C6 alkylene structure,
R₁ and R₂ are each independently a single bond, -O-, or a C1 to C6 alkylene structure,
X₁ and X₂ are each independently SO₃⁻, SO₃⁻M⁺, N(CH₃)₂, N(CH₃)₃⁺Br⁻ or N(C₂H₄OH)₂,
M is H, K, Na or Li, and
m is 0 or 1.

In the definition of Formula 2, specifically, X₁ and X₂ may be each independently SO₃⁻, SO₃⁻M⁺, or a random mixture of SO₃⁻ and SO₃⁻M⁺. That is, the organic hole transporting compound of the present invention has a structure in which a SO₃⁻ functional group is introduced to an end, and therefore excellent solubility in an alcoholic solvent is achieved.

In addition, the organic hole transporting compound may have a P-doped structure in which M⁺ binds to or does not bind to an SO₃⁻ end. Since doping is not performed on the entire compound, there is a mixture of structures in which M⁺ binds and does not bind to an SO₃⁻ end.

For example, in the definition of Formula 2, X₁ and X₂ are the same, and independently SO₃⁻ or SO₃⁻M⁺. In addition, a ratio (D/N) of the number (D) of instances in which X₁ and X₂ are SO₃⁻M⁺ to the number (N) of instances in which X₁ and X₂ are SO₃⁻ may be in a range of 0.1 to 10, 0.1 to 0.9, 1 to 9, 3 to 9, or 3 to 6. The ratio may vary according to a degree of P-doping.

The M may be H, K, Na or Li, and specifically, Na.

In addition, Ar₂ may be represented by Formula 3 below.

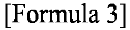

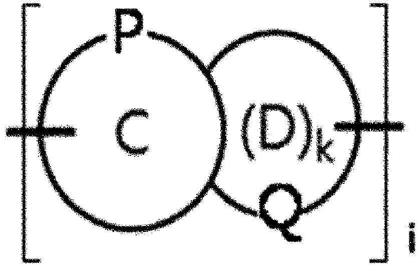

In Formula 3,
C and D are each independently a C3 to C20 ring structure, and include an element represented by P or Q,
k is an integer of 0 or 1, and when k is 0, it represents that there is no ring structure represented by D,
i is an integer of 1 or 2, and
P and Q are each independently any one selected from the group consisting of carbon, oxygen, sulfur and selenium.

Specifically, in the definition of Formula 3,
C and D are each independently a C3 to C20 ring structure, and include an element represented by P or Q,
k is an integer of 0 or 1, and when k is 0, it represents that there is no ring structure represented by D,
i is an integer of 1 or 2, and
P and Q are each independently any one selected from the group consisting of carbon, oxygen, sulfur and selenium.

Hereinafter, detailed structures of Ar₁ and Ar₂ shown in Formula 1 will be specifically examined.

First, examining the detailed structure of Ar₁, as an example, Ar₁₋ may be represented by Formula 2-a below.

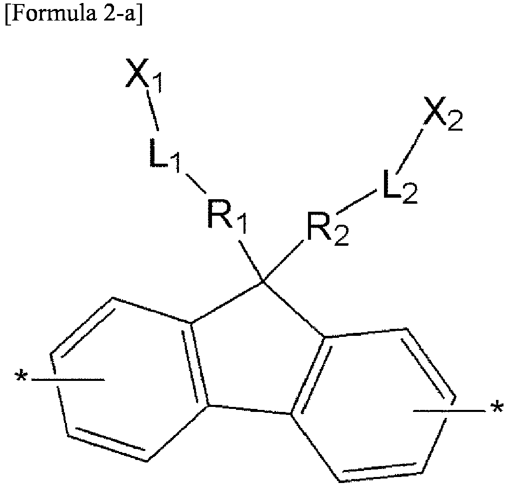

In Formula 2-a,
L₁ and L₂ are each independently a single bond or a C1 to C6 alkylene structure, R₁ and R₂ are each independently a single bond, -O-, or a C1 to C6 alkylene structure,
X₁ and X₂ are each independently SO₃⁻, SO₃⁻M⁺, N(CH₃)₂, N(CH₃)₃⁺Br⁻ or N(C₂H₄OH)₂, and
M is H, K, Na or Li.

In another example, Ar₁₋ may be represented by Formula 2-b below.

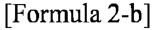

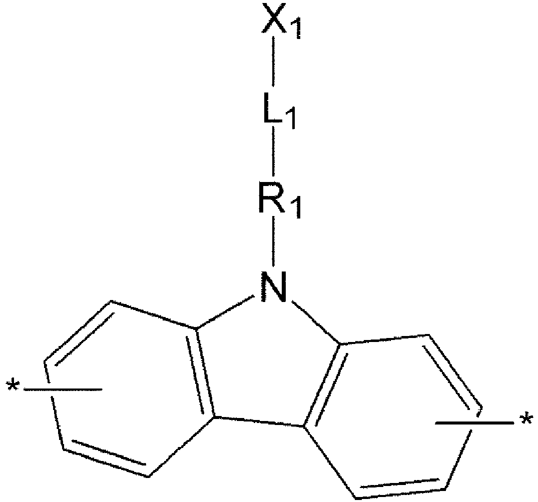

In Formula 2-b,
L₁ is a single bond, or a C1 to C6 alkylene structure,
R₁ is a single bond, -O-, or a C1 to C6 alkylene structure,
X₁ is SO₃⁻, SO₃⁻M⁺, N(CH₃)₂, N(CH₃)₃⁺Br⁻ or N(C₂H₄OH)₂, and
M is H, K, Na or Li.

In still another example, Ar₁₋ may be represented by Formula 2-c below.

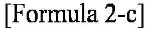

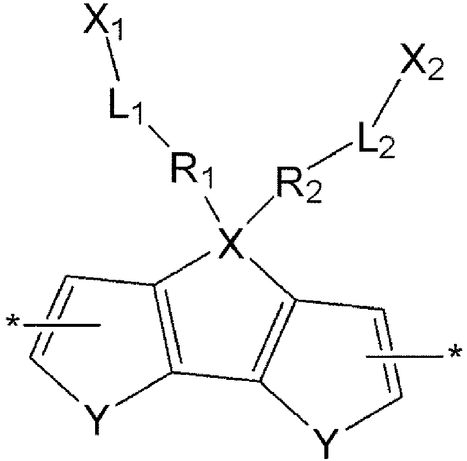

In Formula 2-c,
L₁ and L₂ are each independently a single bond or a C1 to C6 alkylene structure,
R₁ and R₂ are each independently a single bond, -O-, or a C1 to C6 alkylene structure,
X₁ and X₂ are each independently SO₃⁻, SO₃⁻M⁺, N(CH₃)₂, N(CH₃)₃⁺Br⁻ or N(C₂H₄OH)₂,
M is H, K, Na or Li, and
X and Y are each independently carbon, silicon or sulfur.

In yet another example, Ar₁₋ may be represented by Formula 2-d below.

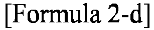

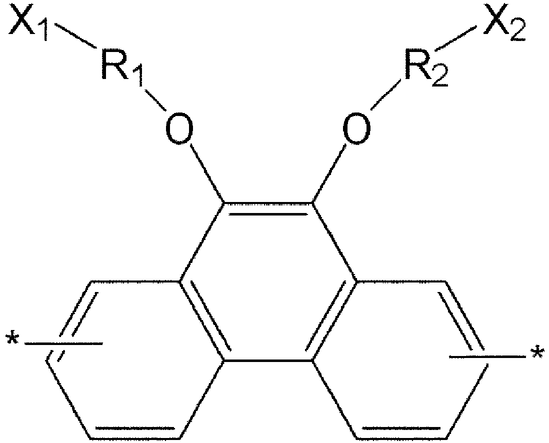

In Formula 2-d,
L₁ and L₂ are each independently a single bond or a C1 to C6 alkylene structure,
R₁ and R₂ are each independently a single bond, -O-, or a C1 to C6 alkylene structure,
X₁ and X₂ are each independently SO₃⁻, SO₃⁻M⁺, N(CH₃)₂, N(CH₃)₃⁺Br⁻ or N(C₂H₄OH)₂, and
M is H, K, Na or Li.

Next, Ar₂ may be represented by any one of Formulas 3-a to 3-c below.

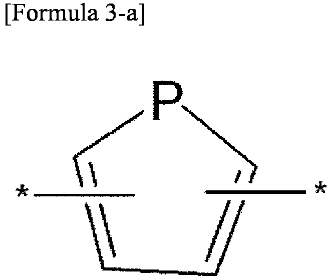

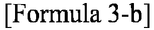

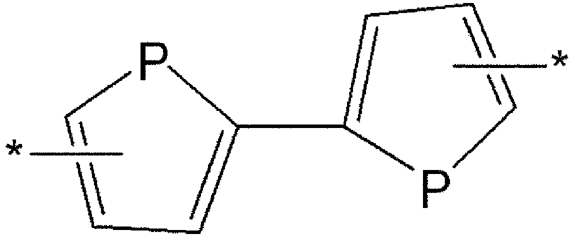

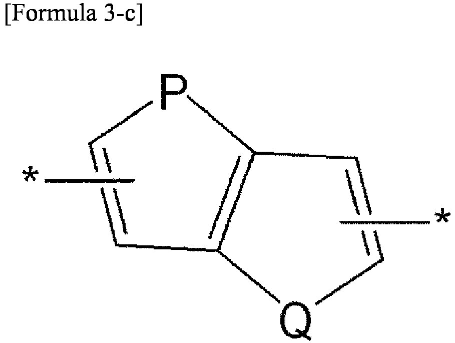

In Formulas 3-a to 3-c,
P and Q are each independently selected from the group consisting of carbon, oxygen, sulfur and selenium.

For example, in Formula 1, Ar₁₋ is selected from structures listed in Table 1 below.

**[Table 1]**

| **No.** | **Ar₁** |
|---|---|
| 1 | 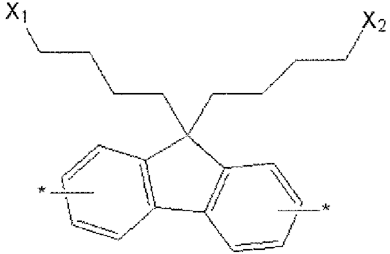 |
| 2 | 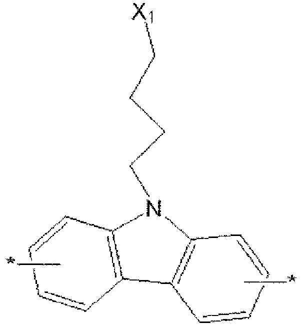 |
| 3 | 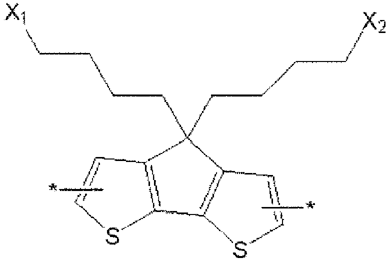 |
| 4 | 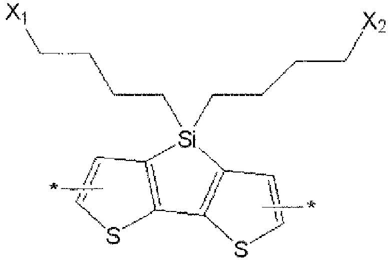 |
| 5 | 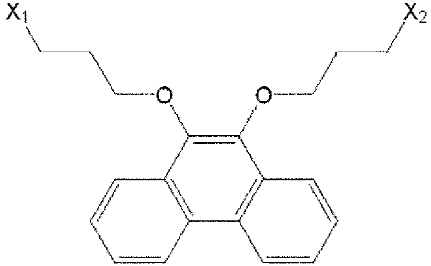 |

In Table 1, X₁ and X₂ are each independently SO₃⁻, SO₃⁻M⁺, N(CH₃)₂, N(CH₃)₃⁺Br⁻ or N(C₂H₄OH)₂, and specifically, SO₃⁻ or SO₃⁻M⁺. In addition, M is H, K, Na or Li, and specifically, Na.

For example, in Formula 1, Ar₂₋ is selected from structures listed in Table 2 below.

**[Table 2]**

| **No.** | **Ar₂** |
|---|---|
| 1 | 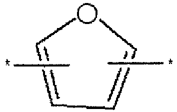 |
| 2 | 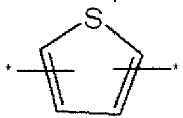 |
| 3 | 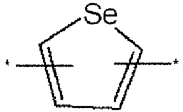 |
| 4 | 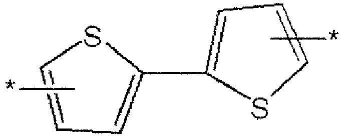 |
| 5 | 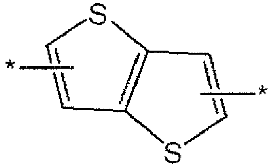 |

The present invention also provides an organic device including the organic hole transporting compound described above, and as the organic device, an OPV is used.

In one example, the OPV includes:
a substrate; a hole transport layer including the organic hole transporting compound described above, which is formed on the substrate; a photoactive layer formed on the hole transport layer; and an electrode formed on the photoactive layer.

The electrode includes, but is not particularly limited to, for example, one or more selected from the group consisting of aluminum (Al), indium tin oxide (ITO), gold (Au), silver (Ag), fluorine-doped tin oxide (FTO), aluminum-doped zinc oxide (AZO), indium zinc oxide (IZO), indium zinc tin oxide (IZTO), ZnO-Ga₂O₃, ZnO-Al₂O₃ and antimony tin oxide (ATO).

In one example, the hole transport layer may include the organic hole transporting compound described above; and a complex including a metal oxide and a precursor of the metal oxide.

The precursor of the metal oxide may be, for example, one or more selected from zinc acetate, titanium(IV) isopropoxide, molybdenum diacetylacetonate dioxide, nickel(II) acetylacetonate, nickel(II) acetate, tungsten(V,VI) ethoxide, phosphomolybdic acid and phosphotungstic acid.

In addition, the metal oxide may be a component prepared by transitioning a precursor of a metal oxide to the metal oxide. For example, the metal oxide may be one or more of zinc oxide, titanium oxide, molybdenum oxide, nickel oxide and tungsten oxide.

In the present invention, for example, a hole transport layer may be introduced at room temperature by spin coating without high temperature thermal treatment. The inventors of the present invention completed an organic solar cell with an inverted structure into which an organic-inorganic complex charge transport layer is introduced using the metal oxide precursor and a conjugated polymer electrolyte material through a low temperature-solution process. They experimentally confirmed that the organic solar cell maintains 70% or higher performance under conditions of room temperature and 30% humidity for 30 days or more without an encapsulation process, by introducing such an organic-inorganic complex to the organic solar cell by means of a hole transport layer. This can replace a conventional deposition process, and exhibits an effect of increasing cost competitiveness and simplifying a process.

In one example, in the hole transport layer, a ratio of the organic hole transporting compound according to the present invention and a total content of the metal oxide and the precursor of the metal oxide may be in a range of 1:10 to 2:1 (based on a weight ratio). The content ratio may be in a range of 1:10 to 1.5:1, 1:5 to 2:1, or 1:5 to 1:1. As the content ratio is controlled, degradation of physical properties of the device can be prevented, and a life span characteristic can be improved.

### [Examples]

Hereinafter, the present invention will be described in further detail with reference to examples. The examples of the present invention are merely provided for detailed explanation of the present invention, and are not intended to limit the scope of the present invention.

### [Preparation Examples 1 to 9] Preparation of precursors of organic hole transporting compounds (1)

In Preparation Examples 1 to 9, precursors of hole transporting compounds represented by Formula 4 below were prepared.

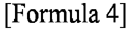

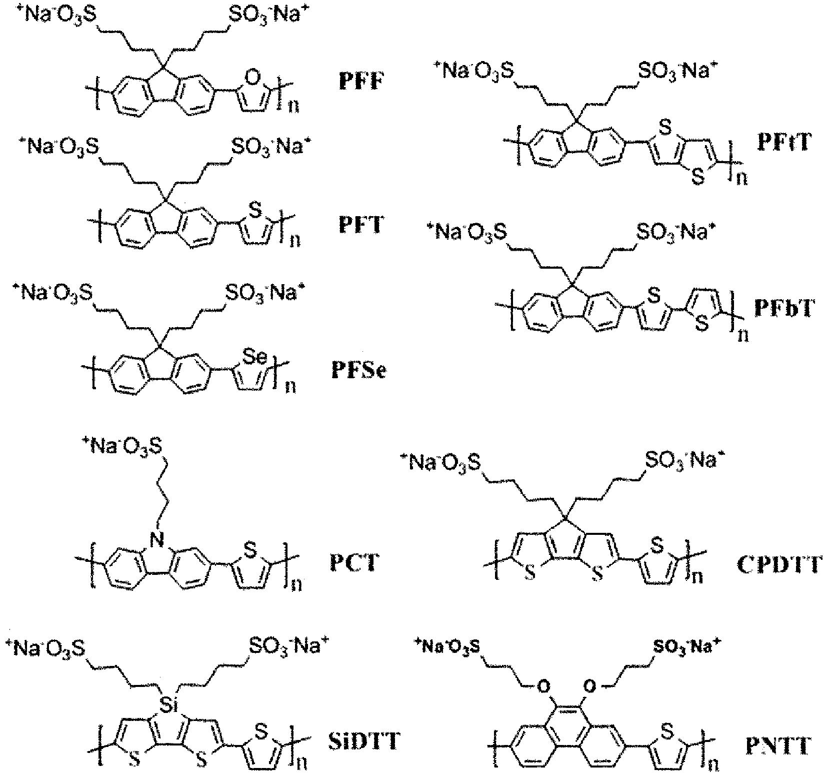

First, Compounds A to E prepared by Reaction Scheme 1 below were obtained.

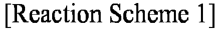

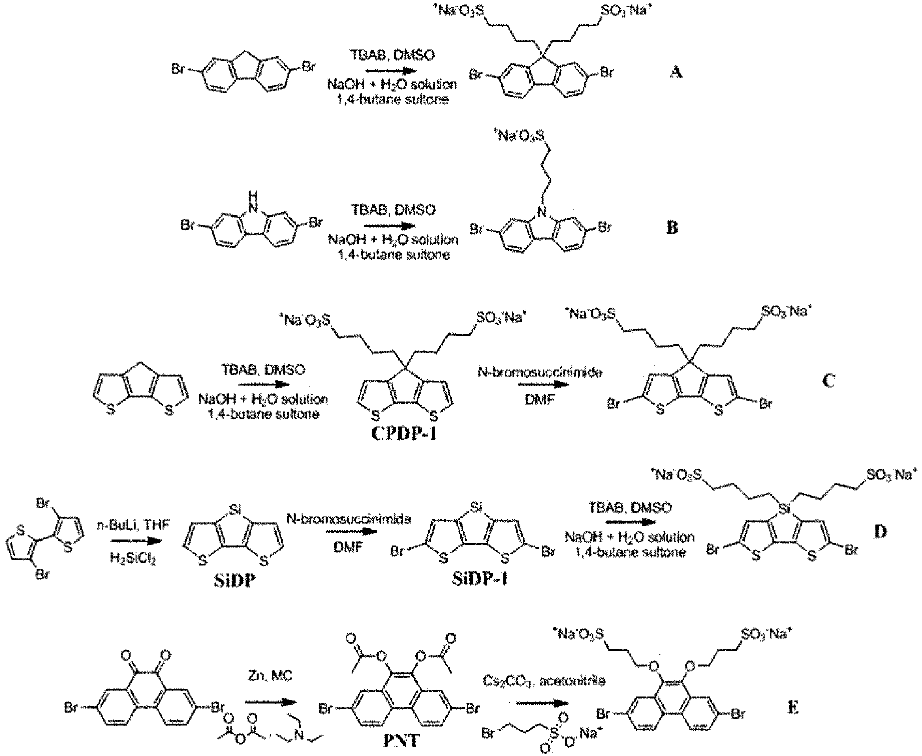

The preparation process will be described in detail.
(1) A mixed solution was prepared by dissolving 3 g of 2,7-dibromo-9H-fluorene and 90 mg of tetrabutylammoniumbromide (TBAB) in 45 ml of dimethyl sulfoxide (DMSO) under a nitrogen atmosphere. An aqueous solution was prepared by dissolving 3 g of sodium hydroxide (NaOH) in 6 ml of distilled water (H₂O), and then slowly added to the mixed solution for 10 minutes or longer, followed by stirring for 30 minutes. Subsequently, 3.15 g of 1,4-butane sultone was added to the stirred solution, and stirred at 100 °C for 12 to 24 hours to perform a reaction. After the reaction, the resulting solution was cooled to room temperature (25 °C), and added to 100 ml of acetone, thereby collecting a precipitate produced after the addition through filtering. The precipitate collected by filtering was washed with acetone several times, thereby obtaining 5.2 g of Compound A (yield: 88.3%).
(2) A mixed solution was prepared by dissolving 1 g of 2,7-dibromo-9H-carbazole and 30 mg of TBAB in 15 ml of DMSO under a nitrogen atmosphere. An aqueous solution was prepared by dissolving 1 g of sodium hydroxide (NaOH) in 2 ml of distilled water (H₂O), and then slowly added to the mixed solution for 10 minutes or longer, followed by stirring for 30 minutes. Subsequently, 1.05 g of 1,4-butane sultone was added to the stirred solution, and stirred at 100 °C for 12 to 24 hours to perform a reaction. After the reaction, the resulting solution was cooled to room temperature (25 °C), and added to 100 ml of acetone, thereby collecting a precipitate produced after the addition through filtering. The precipitate collected by filtering was washed with acetone several times, thereby obtaining 1.27 g of Compound B (yield: 85.2%).
(3) A mixed solution was prepared by dissolving 0.4 g of cyclopenta[2,1-b:3,4-b]dithiophene and 0.02 mg of TBAB in 11 ml of DMSO under a nitrogen atmosphere. 1.5 ml of a 12.5M sodium hydroxide (NaOH) aqueous solution was added to the mixed solution and then stirred. Subsequently, 0.76 g of 1,4-butane sultone was added to the stirred solution, and stirred at 100 °C for 12 hours to perform a reaction. After the reaction, the resulting solution was added to acetone, and a precipitate produced after the addition was filtered, thereby obtaining 0.75 g of Compound CPDT-1 (yield: 71%).
   A mixed solution was prepared by dissolving 0.98 g of the obtained compound CPDT-1 in 11 ml of dimethylformamide. 0.09 g of n-bromosuccinimide was added to the mixed solution, and reacted for 3 hours at room temperature. After the reaction, the resulting solution was added to acetone, and a precipitate produced after the addition was filtered. Powder obtained thereby was purified by reverse-phase column chromatography, thereby obtaining 0.13 g of Compound C (yield: 11%).
(4) A mixed solution was prepared by adding 6.21 g of 3,3'-dibromo-2,2'-bithiophene to 64 ml of anhydrous tetrahydrofuran under a nitrogen atmosphere, and then a temperature was lowered to -78 °C. 15.3 ml of 2.5M n-butyl lithium (n-BuLi) was slowly added dropwise to the prepared mixed solution at -78 °C. The n-BuLi-added mixed solution was stirred for 1 hour at the same temperature, thereby obtaining a white precipitate, and then while 1.94 g of dichlorosilane was slowly added, the resulting mixture was stirred for another five hours at the same temperature. Afterward, the temperature was slowly increased to room temperature, and the mixture was stirred for 16 hours. After a saturated aqueous ammonium chloride solution was added to terminate the reaction, a water-soluble layer was extracted with ether, and then an organic layer was washed with water. The organic layer was dehydrated with anhydrous magnesium sulfate (MgSO₄) and filtered, a solvent was removed, and silica gel column chromatography was performed so as to obtain 1.86 g of silylene-2,2'-bithiophene (SiDT) (yield: 50%).
   1.86 g of the obtained compound SiDT was dissolved in 76.6 ml of dimethylformamide. 3.75 g of n-bromosuccinimide was added, and then a reaction was performed at room temperature for 10 minutes. 100 ml of water was added to terminate the reaction, the mixture was extracted with ether (100 ml), the solvent was removed, and then 3.04 g of 5,5'-dibromo-silylene-2,2'-bithiophene (SiDT-1) (yield: 90%) was obtained through silica gel column chromatography.
   A mixed solution was prepared by dissolving 3.04 g of the obtained SiDT-1 and 83.4 mg of TBAB in 42 ml of DMSO. An aqueous solution of 2.79 g of sodium hydroxide (NaOH) and 5.6 ml of water, which was prepared in another reactor, was added to the mixed solution and stirred. 2.93 g of 1,4-butane sultone was added to the stirred solution, stirred at 100 °C for 12 hours to perform a reaction. After the reaction, acetone was added to the resulting solution, and a precipitate produced after the addition was filtered, thereby obtaining 2.31 g of Compound SiDT-1 (yield: 40%).
(5) A mixed solution was prepared by mixing 0.84 g of 2,7-dibromophenanthrene-9,10-dione, 30 ml of methyl chloride and 1.5 g of zinc (Zn) dust under a nitrogen atmosphere. 2.2 ml of acetic anhydride and 4.8 ml of triethylamine were rapidly added to the mixed solution using a syringe, and stirred at room temperature for 24 hours. The stirred mixed solution was filtered using Celite, washed with a 1M hydrochloric acid (HCl) aqueous solution and 100 mL of a saturated sodium hydrogen carbonate (NaHCO₃) aqueous solution, and extracted with methyl chloride. The methyl chloride was recrystallized with ethanol, thereby obtaining 0.59 g of 2,7-dibromophenanthrene-9,10-diyl diacetate (PNT) (yield: 57%).

A mixed solution was prepared by mixing 0.84 g of the obtained compound PNT, 6.12 g of cesium carbonate and 30 ml of acetonitrile. 4.2 g of sodium 3-bromopropane-1-sulfonate was added to the mixed solution, and a temperature was increased to 75 °C to perform a reaction for 72 hours. When an intermediate was completely consumed in the reaction, the temperature was reduced to room temperature (25 °C), and acetonitrile was removed from the resulting mixed solution using a rotary evaporator. The mixed solution from which the acetonitrile was removed was extracted with methyl chloride and washed with distilled water. An organic phase obtained through extraction and washing was purified with hexane:methyl chloride as an eluent, thereby obtaining 0.98 g of Compound E (yield: 80%).

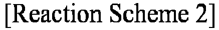

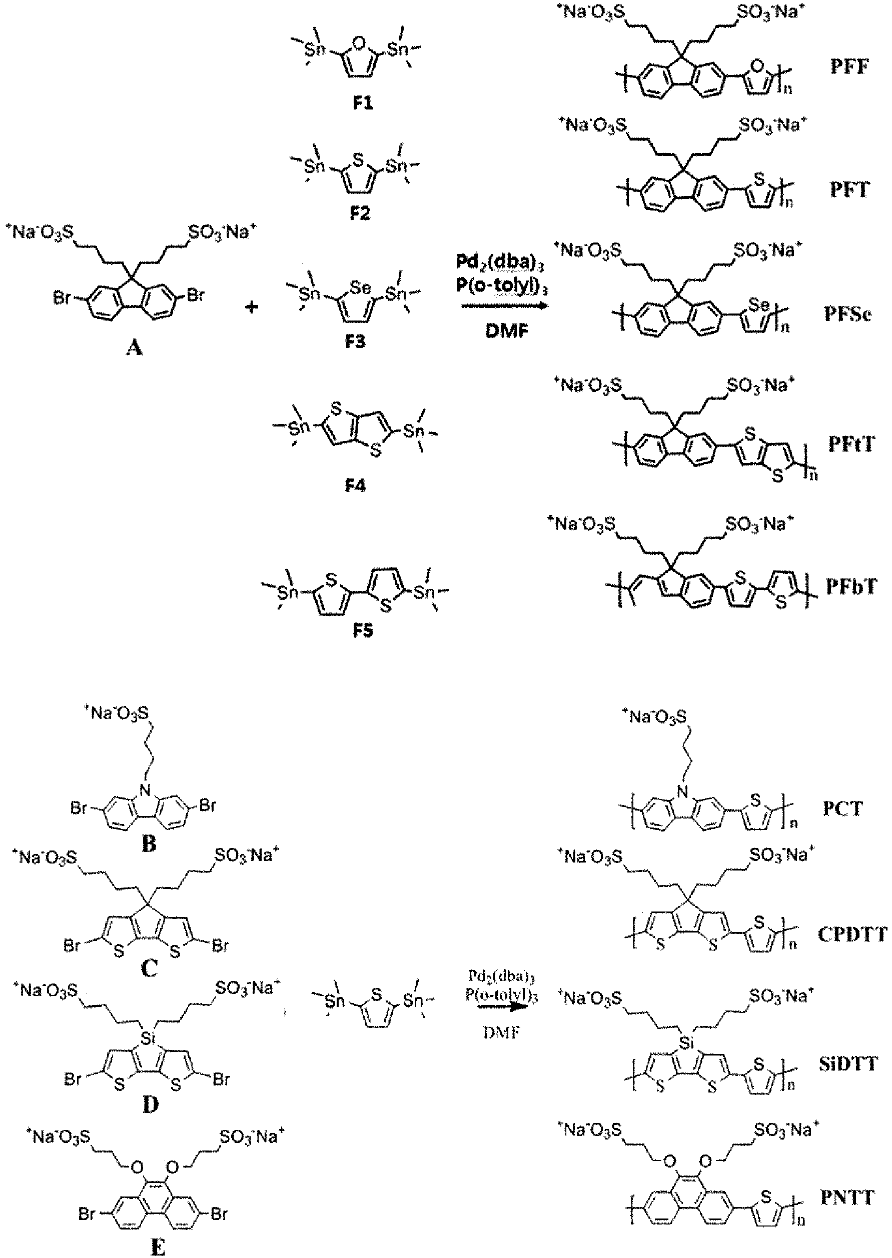

Using Compounds A to E prepared above, precursors of the hole transporting compounds shown in Formula 4 were prepared, respectively. Specifically, the preparation is as follows.

### [Preparation Example 1]

A mixed solution was prepared by dissolving 1.28 g of Compound A and 0.79 g of Compound F1 in 54 ml of dry dimethylformamide (DMF) under a nitrogen atmosphere, and stirring the mixture for 15 minutes or longer. 92 mg of tris(dibenzylideneacetone)dipalladium(0) and 0.12 g of tri-(o-toyl)phosphine) were added to the mixed solution, heated to 100 °C, and stirred for 48 hours or longer to perform a reaction. After the reaction, the resulting mixture was poured into 300 ml of cold acetone to reprecipitate, and the precipitate produced thereby was filtered. The precipitate produced thereby was washed with acetone twice or more, thereby preparing 0.6 g of Compound PFF (yield: 55.0%).

### [Preparation Example 2]

1.0 g of Compound PFT (yield: 88.5%) was prepared by the same method as described in Preparation Example 1, except that 0.82 g of Compound F2 was used instead of Compound F1.

### [Preparation Example 3]

0.31 g of Compound PFSe (yield: 25.0%) was prepared by the same method as described in Preparation Example 1, except that 0.91 g of Compound F3 was used instead of Compound F1.

### [Preparation Example 4]

0.95 g of Compound PFtT (yield: 73.1%) was prepared by the same method as described in Preparation Example 1, except that 0.94 g of Compound F4 was used instead of Compound F1.

### [Preparation Example 5]

0.95 g of Compound PFbT (yield: 70.5%) was prepared by the same method as described in Preparation Example 1, except that 0.98 g of Compound F5 was used instead of Compound F1.

### [Preparation Example 6]

0.57 g of Compound PCT (yield: 65.3%) was prepared by the same method as described in Preparation Example 2, except that 0.96 g of Compound B was used instead of Compound A.

### [Preparation Example 7]

0.71 g of Compound CPDTT (yield: 58.6%) was prepared by the same method as described in Preparation Example 2, except that 1.30 g of Compound C was used instead of Compound A.

### [Preparation Example 8]

0.61 g of Compound SiDTT (yield: 49.5%) was prepared by the same method as described in Preparation Example 2, except that 1.30 g of Compound D was used instead of Compound A.

### [Preparation Example 9]

0.53 g of Compound PNTT (yield: 43.7%) was prepared by the same method as described in Preparation Example 2, except that 1.31 g of Compound E was used instead of Compound A.

### [Examples 1 to 9] Preparation of hole transporting compound

In the present invention, an organic hole transporting compound represented by Formula 5 was prepared.

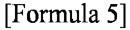

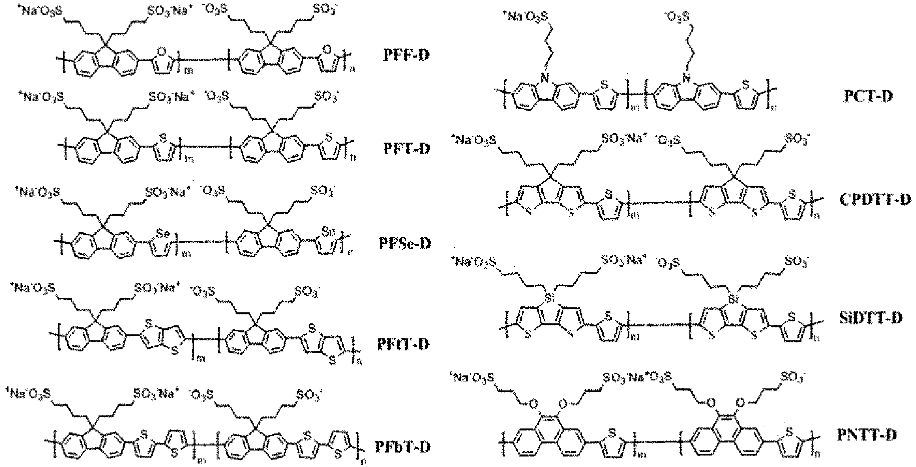

Specifically, organic hole transporting compounds may be prepared by Reaction Scheme 3 using the compounds of Formula 4 (PFF, PFT, PFSe, PFtT, PFbT, PCT, CPDTT, SiDTT, and PNTT), which were prepared by Reaction Scheme 2.

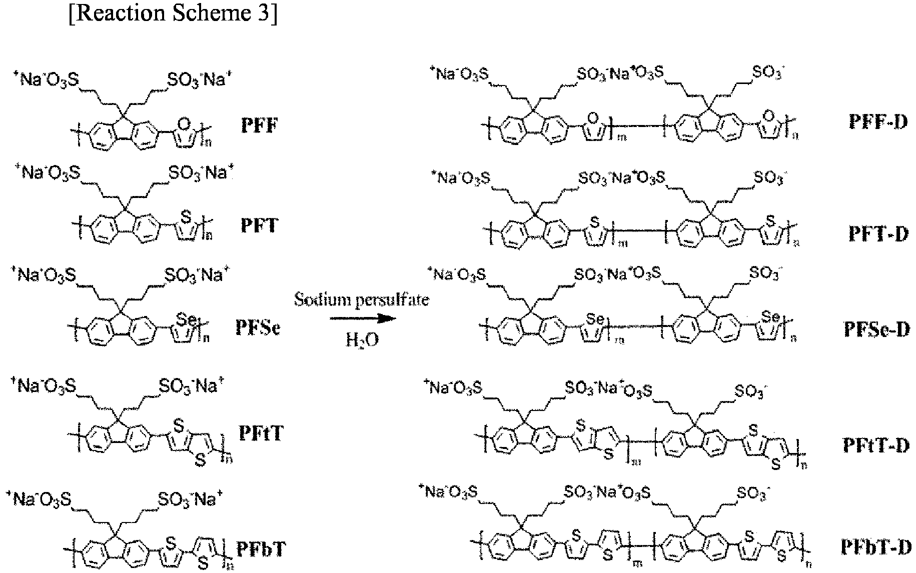

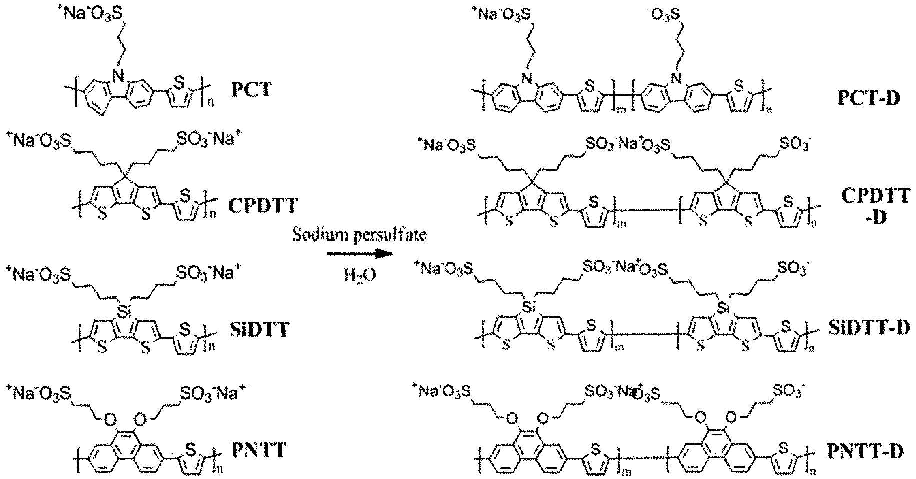

For reference, in Formula 5 and Reaction Scheme 3, although it is illustrated that the instances in which the ends of the main chain of a polymer are SO₃⁻Na⁺ and the instances in which the ends of the main chain of a polymer are SO₃⁻ are repeated in ratios of m and n, respectively, it is only illustrative. Specifically, in Formula 5 and Reaction Scheme 3, the instances in which the ends of the main chain of a polymer are SO₃⁻Na⁺ and the instances in which the ends of the main chain of a polymer are SO₃⁻ are randomly mixed. That is, when the compound in which the ends of the main chain are SO₃⁻Na⁺, is P-doped, Na⁺ is irregularly detached. Theoretically, in the compound in which the ends of the main chain of the molecule are SO₃⁻Na⁺, every Na⁺ is not detached due to P-doping.

### [Example 1]

A mixed solution was prepared by adding an aqueous solution prepared by dissolving 5.71 g of sodium persulfate in 10 ml of distilled water (H₂O) to a solution prepared by dissolving 40 mg of PFF, which was the compound prepared in Preparation Example 1, in 8 ml of distilled water (H₂O). The mixed solution was reacted for 3 hours or longer, reprecipitated by pouring into 300 ml of cold acetone and filtered, thereby collecting a solid. A hole transporting compound PFF-D was prepared by collecting only a part of the collected solid, which was dissolved in ethanol.

### [Example 2]

PFT-D was prepared by the same method as described in Example 1, except that, instead of the PFF compound, 40 mg of the compound prepared in Preparation Example 2, PFT, was used.

### [Example 3]

PFSe-D was prepared by the same method as described in Example 1, except that, instead of the PFF compound, 40 mg of the compound prepared in Preparation Example 3, PFSe, was used.

### [Example 4]

PFtT-D was prepared by the same method as described in Example 1, except that, instead of the PFF compound, 40 mg of the compound prepared in Preparation Example 4, PFtT, was used.

### [Example 5]

PFbT-D was prepared by the same method as described in Example 1, except that, instead of the PFF compound, 40 mg of the compound prepared in Preparation Example 5, PFbT, was used.

### [Example 6]

PCT-D was prepared by the same method as described in Example 1, except that, instead of the PFF compound, 40 mg of the compound prepared in Preparation Example 6, PCT, was used.

### [Example 7]

CPDTT-D was prepared by the same method as described in Example 1, except that, instead of the PFF compound, 40 mg of the compound prepared in Preparation Example 7, CPDTT, was used.

### [Example 8]

SiDTT-D was prepared by the same method as described in Example 1, except that, instead of the PFF compound, 40 mg of the compound prepared in Preparation Example 8, SiDTT, was used.

### [Example 9]

PNTT-D was prepared by the same method as described in Example 1, except that, instead of the PFF compound, 40 mg of the compound prepared in Preparation Example 9, PNTT, was used.

### [Examples 10 to 18] Manufacture of OPVs

Devices were manufactured using the organic hole transporting compounds prepared by Examples 1 to 9 under the following conditions, respectively.

### [Example 10]

The organic hole transporting compound prepared in Example 1 was dissolved in methanol, and filtered using a 0.2-µm PTFE syringe filter. A pretreated ITO substrate and the filtered solution were transferred to a glove box, and then the ITO substrate was spin-coated with the solution to a thickness of 5 nm at 4000 rpm for 30 seconds. After spin coating, the coated ITO substrate was thermally treated at 120 °C for 10 minutes, and the remaining solvent was removed, thereby forming a hole transport layer. The hole transport layer was spin-coated with a photoactive layer solution consisting of polymer (PTB7):PCBM, thereby forming a photoactive layer. Afterward, an Al (5 Å/s, 200 nm) electrode was formed on the photoactive layer using a high vacuum chamber (1×10⁻⁶ Torr or less), thereby manufacturing an OPV.

### [Example 11]

An OPV was manufactured by the same method as described in Example 10, except that, instead of the organic hole transporting compound prepared in Example 1, the hole transporting compound prepared in Example 2 was used.

### [Example 12]

An OPV was manufactured by the same method as described in Example 10, except that, instead of the organic hole transporting compound prepared in Example 1, the hole transporting compound prepared in Example 3 was used.

### [Example 13]

An OPV was manufactured by the same method as described in Example 10, except that, instead of the organic hole transporting compound prepared in Example 1, the hole transporting compound prepared in Example 4 was used.

### [Example 14]

An OPV was manufactured by the same method as described in Example 10, except that, instead of the organic hole transporting compound prepared in Example 1, the hole transporting compound prepared in Example 5 was used.

### [Example 15]

An OPV was manufactured by the same method as described in Example 10, except that, instead of the organic hole transporting compound prepared in Example 1, the hole transporting compound prepared in Example 6 was used.

### [Example 16]

An OPV was manufactured by the same method as described in Example 10, except that, instead of the organic hole transporting compound prepared in Example 1, the hole transporting compound prepared in Example 7 was used.

### [Example 17]

An OPV was manufactured by the same method as described in Example 10, except that, instead of the organic hole transporting compound prepared in Example 1, the hole transporting compound prepared in Example 8 was used.

### [Example 18]

An OPV was manufactured by the same method as described in Example 10, except that, instead of the organic hole transporting compound prepared in Example 1, the hole transporting compound prepared in Example 9 was used.

### Comparative Example 1. Manufacture of OPVs using PEDOT:PSS

PEDOT:PSS was filtered using a 0.45-µm PTFE syringe filter, and stirred in a mixer to prevent phase separation of PEDOT and PSS. A pretreated ITO substrate and a solution prepared by filtering and dissolving the PEDOT:PSS were transferred to a glove box, and the ITO substrate was spin-coated with the solution to a thickness of 30 to 40 nm at 4000 rpm for 30 seconds. After spin coating, the ITO substrate was thermally treated 140 °C for 10 minutes to remove the remaining solvent, thereby forming a hole transport layer. The hole transport layer was spin-coated with a photoactive layer solution consisting of polymer (PTB7):PCBM, thereby forming a photoactive layer. Afterward, an Al (5 Å/s, 200 nm) electrode was formed on the photoactive layer using a high vacuum chamber (1×10⁻⁶ Torr or less) of a thermal evaporator.

### Experimental Example 1

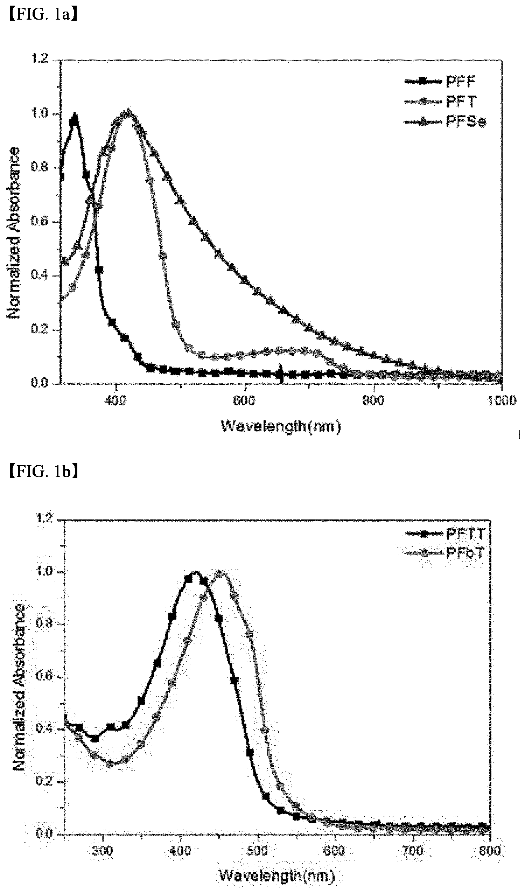

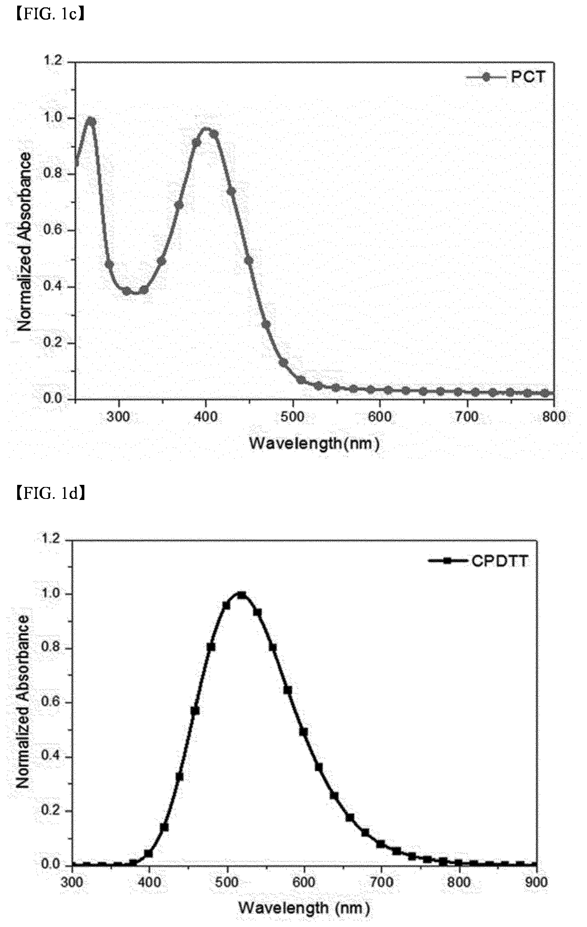

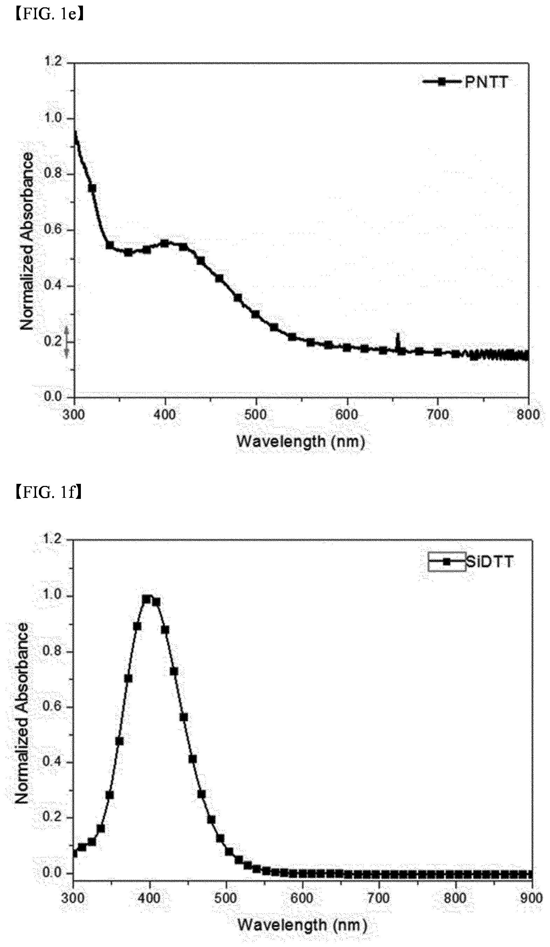

To evaluate absorbances of a precursor of the organic hole transporting compound and the organic hole transporting compound according to the present invention, absorbances using UV-Vis spectra were measured for the precursors prepared in Preparation Examples 1 to 9 and the compounds prepared in Examples 1 to 9, and the measured results are shown in FIGS. 1A to IF and FIGS. 2A to 2E.

FIGS. 1A to IF show the results of measuring absorbances of UV-Vis spectra for precursors prepared in Preparation Examples 1 to 9.

FIGS. 2A to 2E are the results of measuring UV-Vis spectra of the compounds of Examples 1 to 5 by changing conditions such as a reaction time and molarity in a sodium persulfate solution. As seen from the graphs, it can be confirmed that, as the reaction time with sodium persulfate increases, (a doping time increases), peak shifts towards a shorter wavelength.

From such a result, it can be confirmed that an absorption region of the organic hole transporting compound according to the present invention is shifted toward a shorter wavelength as a reaction time increases, and the reaction progresses as much as possible until a region of the absorption wavelength does not shift any more. In addition, it can be seen that, due to the shift of the absorption wavelength region of the compound towards a shorter wavelength, when the compound is applied to an interlayer such as an HTL, the loss of sunlight introduced into a solar cell photoactive layer is small.

### Experimental Example 2

FIGS. 3A to 3C show absorbance graphs and graphs of PL characteristics when three polymers of PFT, PFtT and PFbT, which are the compounds represented by Formula 4, were converted into PFT-D, PFtT-D and PFbT-D, which are the compounds represented by Formula 5, respectively.

The left graphs of FIGS. 3A to 3C are absorbance graphs. While FIGS. 2A to 2E show the change in absorption regions to confirm degrees of doping of PFT, PFtT and PFbT, the absorbance graphs of FIGS. 3A to 3C show the comparison of the absorbances of the compounds represented by Formula 4, which are PFT, PFtT and PFbT, and the compounds represented by Formula 5, which are PFT-D, PFtT-D and PFbT-D, after collection of the completely-doped PFT-D, PFtT-D, PFbT-D polymers. Since the absorption regions of PFT-D, PFtT-D and PFbT-D are shorter wavelength regions than PFT, PFtT and PFbT (the regions containing less photon energy from sunlight reaching the earth surface), most of the sunlight passes through the photoactive layer, which is preferable for energy harvesting.

The right graphs of FIGS. 3A to 3C are graphs of PL characteristics, in which PFT-D, PFtT-D and PFbT-D show absorption characteristics in a shorter wavelength region than PFT, PFtT and PFbT. Among these polymers, photoluminescence peaks of the two polymers excluding PFtT were also observed in a shorter wavelength region. PFtT showed an insignificant shift of the absorption region towards a short wavelength after doping, compared to other two polymers, and therefore, there was no significant difference in a photoluminescence peak before and after doping.

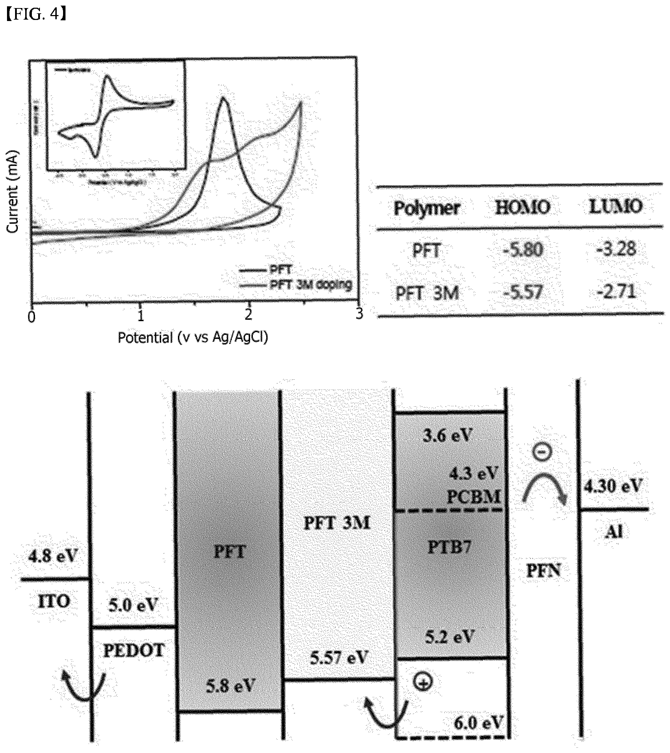

FIG. 4 shows energy levels of the PFT polymers, measured before and after doping. In FIG. 4, the energy Venn diagram shown below is made using data measured to compare the work function of PEDOT:PSS and HOMO levels of the PFT polymers, when the polymers are applied to a solar cell device. It is ideal that the work function of an HTL is present between the HOMO, -5.27eV, of PTB7 used as a photoactive layer and the work function, -4.8eV, of an ITO electrode. PEDOT:PSS has a work function of -5.0eV in a corresponding region, and the PFT polymers have HOMO levels lower than PTB7, and therefore they are unfavorable in hole mobility, compared to PEDOT:PSS. However, when applied to an actual device, PFT shows a higher PCE value, and therefore it can be seen that a method of moving a hole through a PFT layer may be different from PEDOT:PSS. In the example of the present invention, PEDOT:PSS is formed to a thickness of 30 to 40 nm, but it can be seen that, since the PFT polymer is very thinly introduced at a thickness of 5 nm or less, holes move to an electrode by passing through PFT due to a tunneling effect caused by a dipole moment, not moving along an energy band.

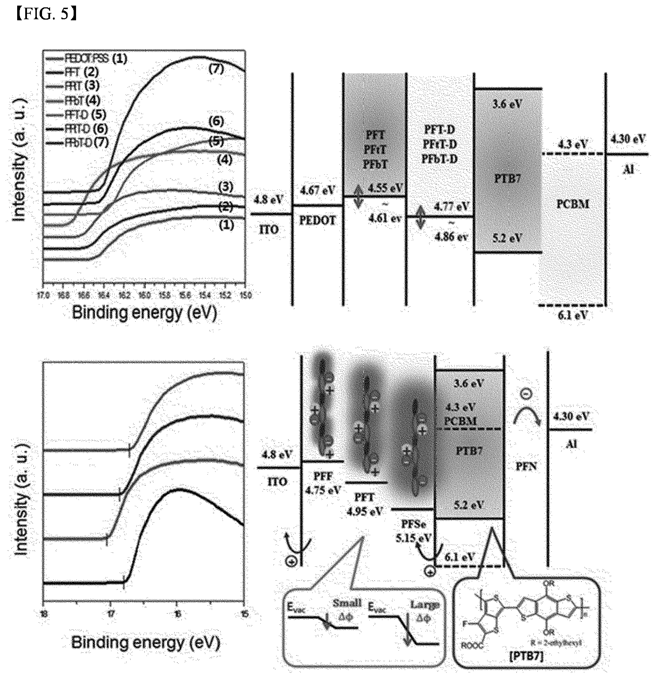

FIG. 5 shows work function values measured by UPS after an ITO glass is spin-coated with PFF, PFT, PFSe, PFtT, PFbT, and PFT-D, PFtT-D and PFbT-D, which are doped with these polymers.

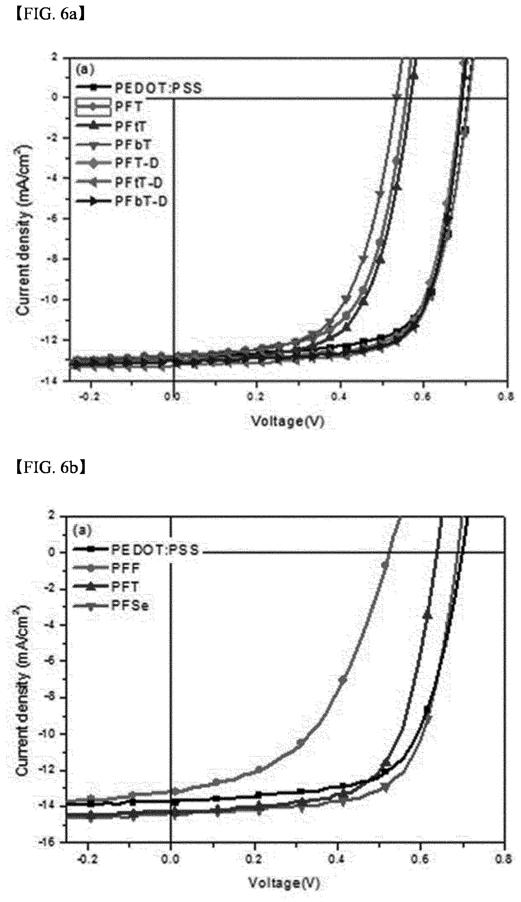

FIGS. 6A and 6B are graphs and tables showing characteristic evaluations when the compounds represented by Formula 4, and the polymers represented by Formula 5 such as PFT-D, PFtT-D, PFbT-D, which are doped with these polymers, are applied to an organic solar cell as an HTL. When predoped five types of polymers such as PFF, PFT, PFSe, PFtT and PFbT and doped polymers were introduced as an HTL to a solar cell, a device is implemented as a battery, and therefore it can be confirmed that the polymers act as a hole transport layer. As compared with PEDOT:PSS, which was introduced as a comparison group, the polymers of Compound C, excluding PFSe, had current densities similar to those of PEDOT:PSS, but were decreased in Voc and FF, thereby exhibiting low PCE. The PFSe, PFT-D, PFtT-D and PFbT-D polymers were increased in current density (Jsc), and improved in PCE value. Accordingly, it can be confirmed that the four polymers such as PFSe, PFT-D, PFtT-D and PFbT-D are materials which can sufficiently replace PEDOT:PSS.

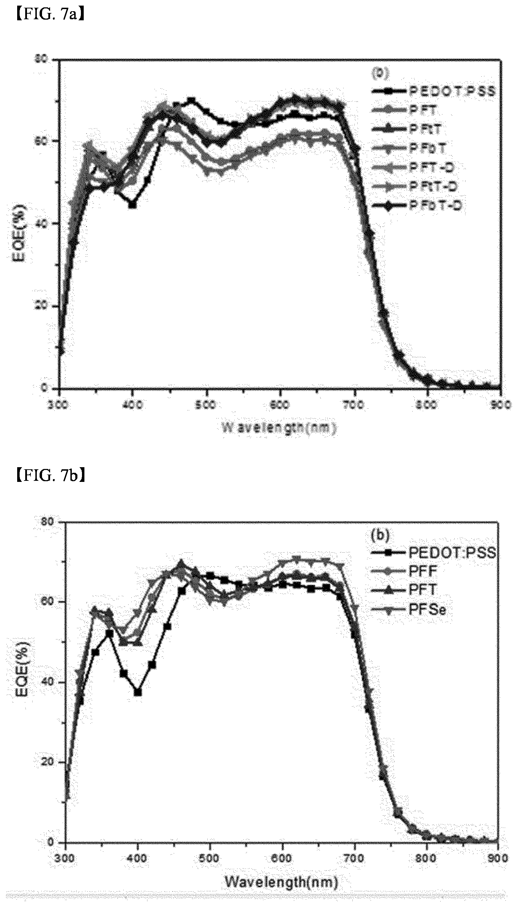

FIGS. 7A and 7B show the results of measuring EQE when each of the compounds represented by Formula 4 and the polymers represented by Formula 5, which are doped with those of Formula 4, for example, PFT-D, PFtT-D and PFbT-D was applied as an HTL to an organic solar cell. The compounds represented by Formula 4 and the polymers represented by Formula 5, which are doped with those of Formula 4, for example, PFT-D, PFtT-D and PFbT-D have higher EQEs than PEDOT:PSS at a short wavelength of 450 nm or less. This indicates that, when PEDOT:PSS is introduced as an HTL, the quantity of electric charge generated by absorbing light with a wavelength of 400 nm is small. In addition, when the PFSe, PFT-D, PFtT-D and PFbT-D polymers are applied as an HTL, it can be confirmed that the EQE is increased even to a very small extent in a wavelength range of 550 to 700 nm. Therefore, it can be confirmed that the PFSe, PFT-D, PFtT-D and PFbT-D polymers have high Jsc values.

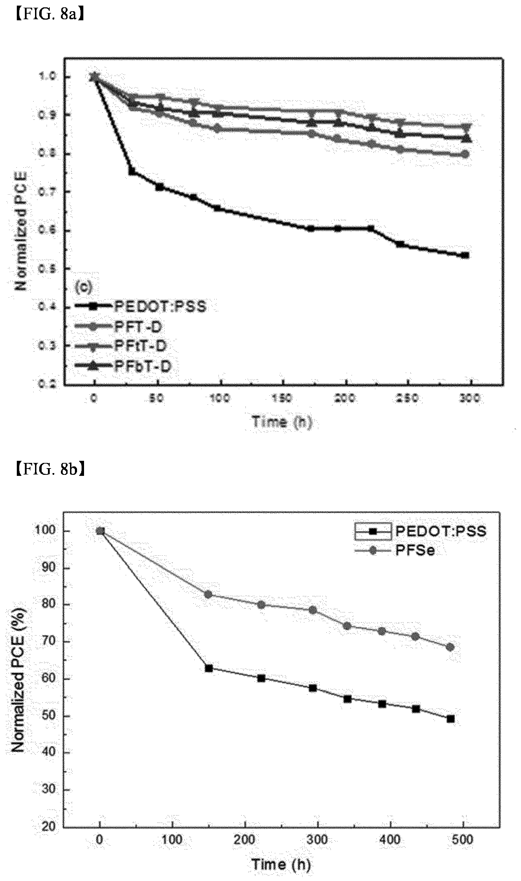

FIGS. 8A and 8B are long-term stability test results for organic solar cells using the PFT-D, PFtT-D, PFbT-D and PFSe polymers according to the present invention and an organic solar cell using PEDOT:PSS. It can be confirmed that an organic solar cells using the PFT-D, PFtT-D, PFbT-D or PFSe polymer dissolved in an alcohol is less decreased in efficiency than PEDOT:PSS, which is acidic in a solution, for the same time. That is, the organic solar cells using the PFT-D, PFtT-D, PFbT-D and PFSe polymers have more excellent long-term stability.

### [Example 19] Manufacture of OPVs

Each of the organic hole transporting compound PFSe-D, prepared in Example 3, and molybdenum diacetylacetonate dioxide was dissolved in methanol, and stirred at 60 °C for 1 hour or longer. The molybdenum diacetylacetonate dioxide and the stirred PFSe solution were mixed at a molar ratio of 1:0.2, 1:0.4, 1:0.6, 1:0.8 and 1:1. Afterward, to improve a film forming property on an organic photoactive layer, the mixed solution of the molybdenum diacetylacetonate dioxide and PFSe-D was mixed with 1-butanol in a volume ratio of 9:1, 7:3 or 5:5, thereby preparing a hole transport layer solution.

An OPV was prepared by the same method as described in Example 10, except that, instead of the organic hole transporting compound prepared in Example 1, the hole transporting compound prepared above was used.

### Experimental Example 3

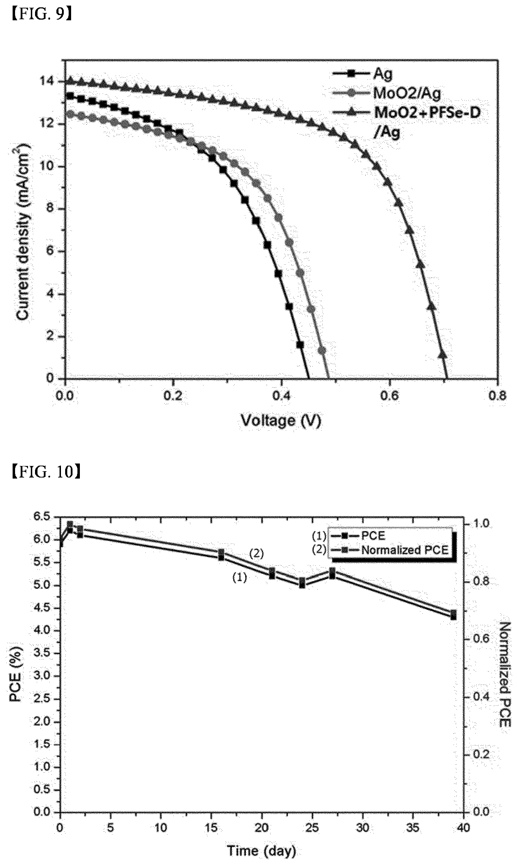

FIG. 9 is a voltage-current density graph for organic solar cells in which molybdenum diacetylacetonate dioxide (MoO₂(acac)₂) and MoO₂+PFSe-D are introduced into an HTL. In FIG. 9, the case in which only Ag was introduced is Comparative Example 2, and the case in which MoO₂/Ag was introduced is Comparative Example 3.

FIG. 10 shows the result of evaluating the long-term stability for an organic solar cell in which MoO₂ + PFSe-D according to Example 19 is introduced as an HTL. FIG. 10 shows the graph plotted by measuring the change in energy conversion efficiency of an organic solar cell device according to time for 40 days while the device is maintained in an atmosphere of 25 °C and a humidity of 30% in a dark room without a separate encapsulation process in order to evaluate the long-term stability of the device.

After one day, the manufactured organic solar cell device had the maximum value of the energy conversion efficiency, that is, 6.2%. After 35 days, it was confirmed that the organic solar cell device also shows excellent long-term stability, which is approximately 70% of the maximum efficiency.

Table 3 shows open circuit voltages, short circuit current densities, fill factors and energy conversion efficiency, which are calculated from the graph of FIG. 9.

**[Table 3]**

| | Short circuit current density Jsc (mA/cm²) | Open circuit voltage Voc (V) | Fill Factor (%) | Energy conversion efficiency (%) |
|---|---|---|---|---|
| Example 19 | 14.1 | 0.6969 | 60.1 | 5.9 |
| Comparative Example 2 | 13.4 | 0.4546 | 47.2 | 2.9 |
| Comparative Example 3 | 12.5 | 0.495 | 52.4 | 3.3 |

Referring to Table 3, it was confirmed that the open circuit voltage and the fill factor of the organic solar cell device (Example 19) into which molybdenum diacetylacetonate dioxide (MoO₂(acac)₂)+PFSe-D was introduced were increased by 53.3% and 21.5%, compared to the devices (Comparative Example 2 and Comparative Example 3) in which this material was not introduced, and therefore the energy conversion efficiency was increased by 103%.

### [Industrial Applicability]

An organic hole transporting compound and an OPV including the same are provided, and the organic hole transporting compound can realize highly efficient hole mobility with excellent long-term stability, and can be effectively applied to the OPV.

## Claims

1. An organic hole transporting compound, comprising a repeat unit represented by Formula 1: 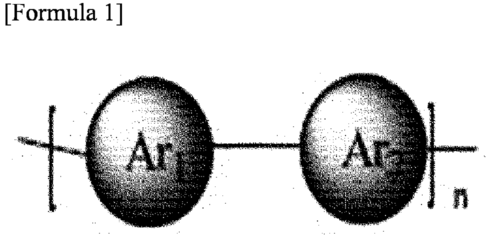 where Ar₁₋ is represented by Formula 2 below,
Ar₂ is represented by Formula 3 below,
n is an integer of 1 to 1,000,000, 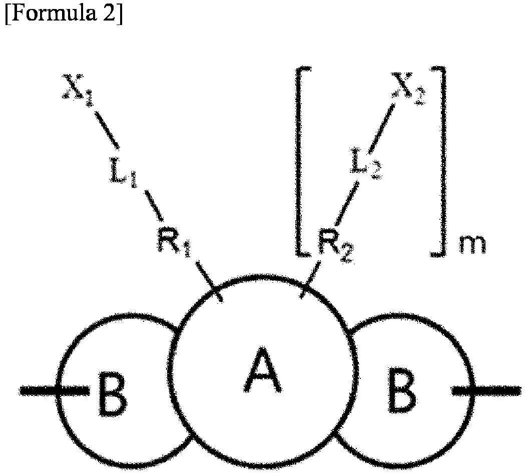 where A is a C3 to C16 cyclic structure, in which any one or more of the carbons forming the cyclic structure A are substituted with N or Si, or not substituted,
B is a ring structure which is linked to the cyclic structure A at both ends,
L₁ and L₂ are each independently a single bond, a C1 to C6 alkylene, C6 to C16 arylene or C3 to C15 heteroarylene structure,
R₁ and R₂ are each independently any one selected from the group consisting of a single bond, a double bond, -O-, C1 to C6 alkylene; C1 to C6 alkoxylene; thiophene; thiophene substituted with a C1 to C25 alkyl group; thiophene substituted with a C1 to C25 alkoxy group; selenophene; selenophene substituted with a C1 to C25 alkyl group; selenophene substituted with a C1 to C25 alkoxy group; pyrrole; pyrrole substituted with a C1 to C25 alkyl group; pyrrole substituted with a C1 to C25 alkoxy group; an arylene group; an arylene group substituted with a C1 to C25 alkyl group; an arylene group substituted with a C1 to C25 alkoxy group; an aryl group; an aryl group substituted with a C1 to C25 alkyl group; an aryl group substituted with a C1 to C25 alkoxy group; thiazole; thiazole substituted with a C1 to C25 alkyl group; thiazole substituted with a C1 to C25 alkoxy group; and a C10 to C24 aryl group having a fused aromatic compound,
X₁ and X₂ are each independently SO₃⁻, SO₃⁻M⁺, N(CH₃)₂, N(CH₃)₃⁺Br⁻ or N(C₂H₄OH)₂,
M is H, K, Na or Li,
m is 0 or 1, 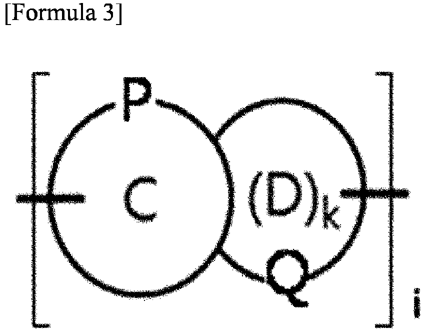 where C and D are each independently a C3 to C20 ring structure, and include an element represented as P or Q,
k is an integer of 0 or 1, and when k is 0, it represents that there is no ring structure represented as D,
i is an integer of 1 or 2, and
P and Q are each independently any one selected from the group consisting of carbon, oxygen, sulfur and selenium.

2. The compound of claim 1, wherein, in the definition of Formula 2,
A is a C3 to C16 cyclic structure, in which any one or more of the carbons forming the cyclic structure A are substituted with N or Si, or not substituted,
B is a ring structure which is linked to the cyclic structure A at both ends,
L₁ and L₂ are each independently a single bond or a C1 to C6 alkylene structure, R₁ and R₂ are each independently a single bond, -O-, or a C1 to C6 alkylene structure, X₁ and X₂ are each independently SO₃⁻, SO₃⁻M⁺, N(CH₃)₂, N(CH₃)₃⁺Br⁻ or N(C₂H₄OH)₂,
M is H, K, Na or Li, and
m is 0 or 1.

3. The compound of claim 1, wherein Ar₁₋ is represented by Formula 2-a below: 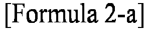 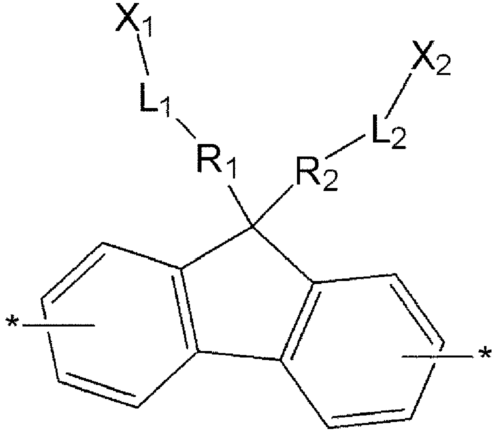 where L₁ and L₂ are each independently a single bond or a C1 to C6 alkylene structure,
R₁ and R₂ are each independently a single bond, -O-, or a C1 to C6 alkylene structure,
X₁ and X₂ are each independently SO₃⁻, SO₃⁻M⁺, N(CH₃)₂, N(CH₃)₃⁺Br⁻ or N(C₂H₄OH)₂, and
M is H, K, Na or Li.

4. The compound of claim 1, wherein Ar₁₋ is represented by Formula 2-b below: 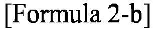 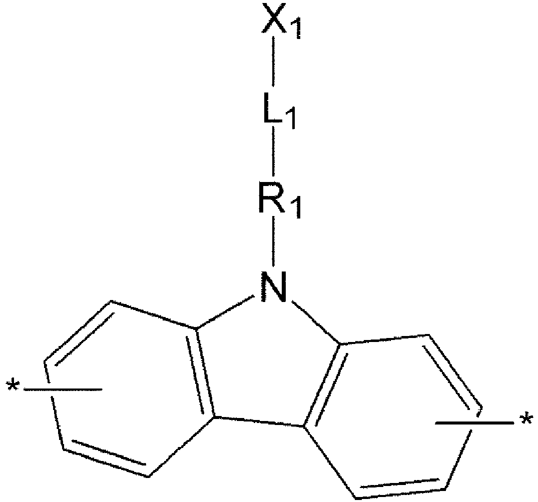 where L₁ is a single bond or a C1 to C6 alkylene structure,
R₁ is a single bond, -O-, or a C1 to C6 alkylene structure,
X₁ is SO₃⁻, SO₃⁻M⁺, N(CH₃)₂, N(CH₃)₃⁺Br⁻ or N(C₂H₄OH)₂, and
M is H, K, Na or Li.

5. The compound of claim 1, wherein Ar₁₋ is represented by Formula 2-c below: 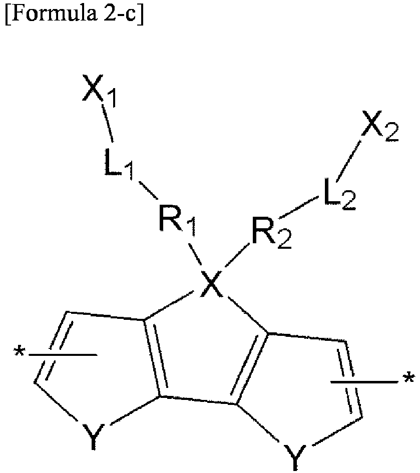 where L₁ and L₂ are each independently a single bond or a C1 to C6 alkylene structure,
R₁ and R₂ are each independently a single bond, -O-, or a C1 to C6 alkylene structure,
X₁ and X₂ are each independently SO₃⁻, SO₃⁻M⁺, N(CH₃)₂, N(CH₃)₃⁺Br⁻ or N(C₂H₄OH)₂,
M is H, K, Na or Li, and
X and Y are each independently carbon, silicon or sulfur.

6. The compound of claim 1, wherein Ar₁₋ is represented by Formula 2-d below: 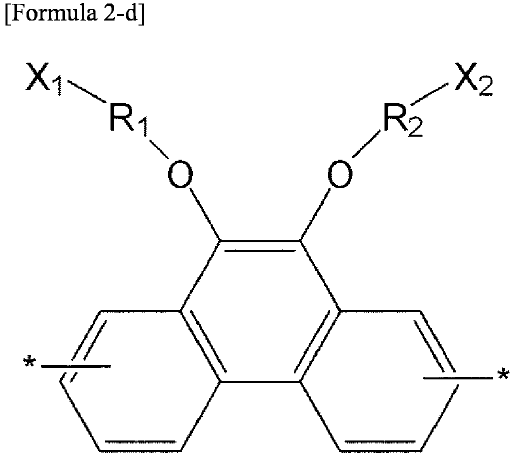 where L₁ and L₂ are each independently a single bond or a C1 to C6 alkylene structure,
R₁ and R₂ are each independently a single bond, -O-, or a C1 to C6 alkylene structure,
X₁ and X₂ are each independently SO₃⁻, SO₃⁻M⁺, N(CH₃)₂, N(CH₃)₃⁺Br⁻ or N(C₂H₄OH)₂, and
M is H, K, Na or Li.

7. The compound of claim 1, wherein Ar₂ is presented by any one of Formulas 3-a to 3-c below: 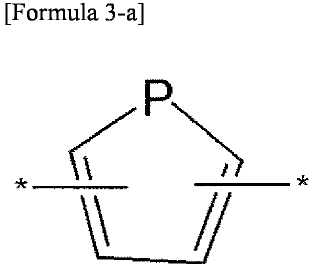 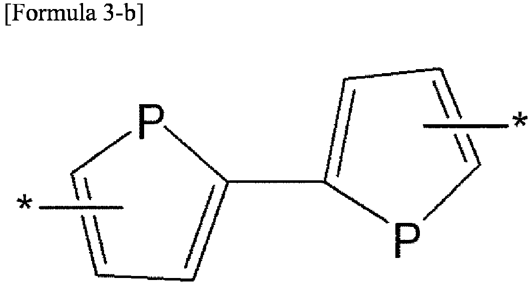 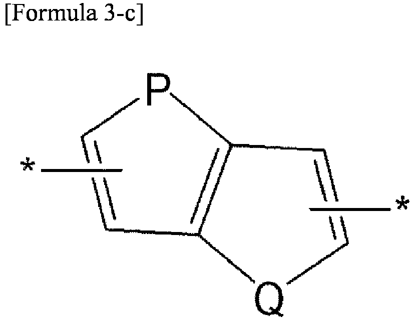 where P and Q are each independently selected from the group consisting of carbon, oxygen, sulfur and selenium.

8. The compound of claim 1, wherein, in the definition of Formula 2,
X₁ and X₂ are each independently SO₃⁻ or SO₃⁻M⁺, and
M is H, K, Na or Li.

9. The compound of claim 1, wherein, in the definition of Formula 2,
X₁ and X₂ are the same, and SO₃⁻ or SO₃⁻M⁺,
M is H, K, Na or Li, and
a ratio (D/N) of the number (D) of instances in which X₁ and X₂ are SO₃⁻M⁺ to the number (N) of instances in which X₁ and X₂ are SO₃⁻ is in a range of 0.1 to 10.

10. The compound of claim 1, wherein Ar₁₋ is a structure represented in Table 1 below, X₁ and X₂ are each independently SO₃⁻ or SO₃⁻M⁺, and
M is H, K, Na or Li.
**[Table 1]**
| **No.** | **Ar₁** |
|---|---|
| 1 | 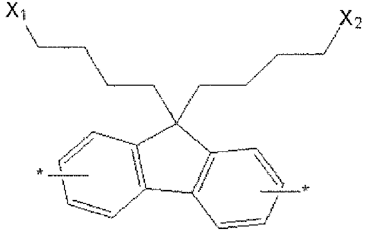 |
| 2 | 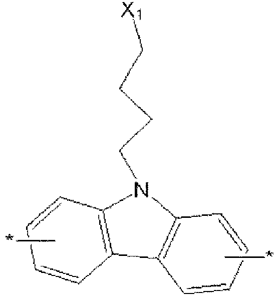 |
| 3 | 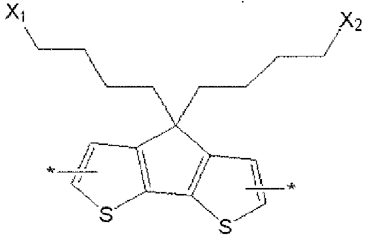 |
| 4 | 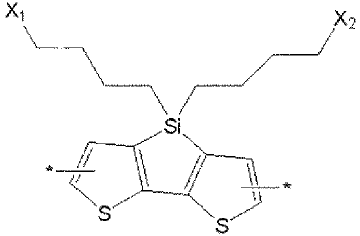 |
| 5 | 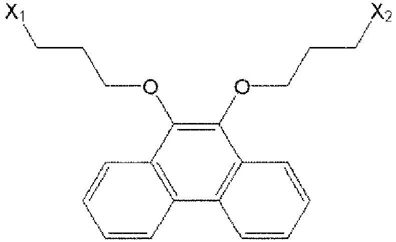 |

11. The compound of claim 1, wherein Ar₂ is a structure represented in Table 2 below.
**[Table 2]**
| **No.** | **Ar₂** |
|---|---|
| 1 | 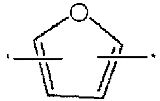 |
| 2 | 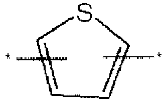 |
| 3 | 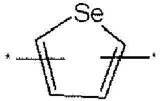 |
| 4 | 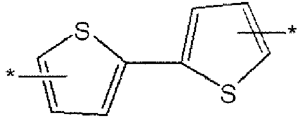 |
| 5 | 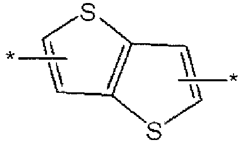 |

12. An organic photovoltaic device comprising the organic hole transporting compound according to any one of claims 1 to 11.

13. The device of claim 12, wherein the organic photovoltaic device comprises:
a substrate;
a hole transport layer comprising the organic hole transporting compound according to any one of claims 1 to 10, which is formed on the substrate;
a photoactive layer formed on the hole transport layer; and
an electrode formed on the photoactive layer.

14. The device of claim 13, wherein the electrode comprises one or more selected from the group consisting of aluminum (Al), indium tin oxide (ITO), gold (Au), silver (Ag), fluorine-doped tin oxide (FTO), aluminum-doped zinc oxide (AZO), indium zinc oxide (IZO), indium zinc tin oxide (IZTO), ZnO-Ga₂O₃, ZnO-Al₂O₃ and antimony tin oxide (ATO).

15. The device of claim 13, wherein the hole transport layer comprises:
the organic hole transporting compound according to any one of claims 1 to 11; and
a metal oxide and a precursor of the metal oxide.

16. The device of claim 15, wherein, in the hole transport layer, a ratio of the organic hole transporting compound according to the present invention and a total content of the metal oxide and the precursor of the metal oxide may be in a range of 1:10 to 2:1 (based on a weight ratio).
